(19) Europäisches Patentamt
European Patent Office
Office européen des brevets

(11) Numéro de publication: **0 051 514**
**B1**

# (12) FASCICULE DE BREVET EUROPEEN

(45) Date de publication du fascicule du brevet: **23.11.83**

(51) Int. Cl.³: **C 07 C 59/84**, C 07 C 59/90, C 07 C 59/88, C 07 C 69/76, C 07 C 69/94

(21) Numéro de dépôt: **81401611.9**

(22) Date de dépôt: **15.10.81**

(54) **Dérivé d'acide cinnamoyl-cinnamique, son procédé de préparation et son application en thérapeutique.**

(30) Priorité: **23.10.80 FR 8022693**

(43) Date de publication de la demande: **12.05.82 Bulletin 82/19**

(45) Mention de la délivrance du brevet: **23.11.83 Bulletin 83/47**

(84) Etats contractants désignés: **AT BE CH DE FR GB IT LI LU NL SE**

(56) Documents cités:
**FR - A - 2 278 332**
**FR - A - 2 383 157**
**US - A - 4 124 724**
**US - A - 4 163 859**

**THE JOURNAL OF ORGANIC CHEMISTRY, vol. 34, no. 3, mars 1969 EASTON, Pa. (US) G.H. CLELAND: "The Meerwein Reaction in Amino Acid Synthesis. II. An Investigation of Twenty-one Substituted Anilines", pages 744-747**

(73) Titulaire: **SOCIETE DE RECHERCHES INDUSTRIELLES S.O.R.I. Société anonyme dite:, 3 rue de Citeaux, F-75012 Paris (FR)**

(72) Inventeur: **Picart, François, 38 rue Devosge, F-21000 Dijon (FR)**

(74) Mandataire: **Clisci, Serge et al, CABINET BEAU DE LOMENIE 55 rue d'Amsterdam, F-75008 Paris (FR)**

**EP 0 051 514 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

Dérivé d'acide cinnamoyl-cinnamique, son procédé de préparation et son application en thérapeutique.

La présente invention concerne en tant que produit industriel nouveau une chalcone qui est un dérivé d'acide cinnamoyl-cinnamique. Elle concerne également son procédé de préparation et son application en thérapeutique notamment en tant qu'agent régulateur de croissance.

On vient de trouver de façon surprenante que les composés de formule I ci-après qui sont des chalcones appartenant à la famille des dérivés d'acide cinnamoyl-cinnamique sont utiles en thérapeutique, en particulier en tant qu'agents régulateurs de croissance. Ils permettent la restauration d'un phénotype cellulaire de cellule normale chez une cellule transformée (par exemple une cellule à croissance anarchique) et sont utiles notamment dans le traitement des maladies liées à un développement anarchique des cellules et en particulier dans le traitement des affections dermatologiques telles que psoriasis, kératose cutanée, acné, eczéma.

Le nouveau dérivé d'acide cinnamoyl-cinnamique selon l'invention est caractérisé en ce qu'il est choisi parmi l'ensemble constitué par

(i) les dérivés d'acide m-cinnamoyl-cinnamique de formule

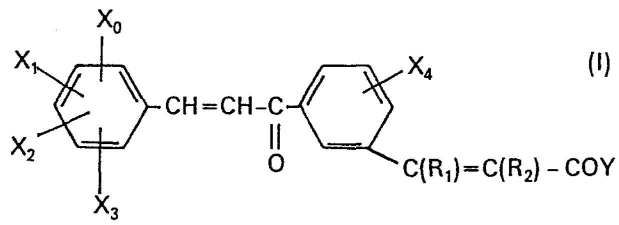

(I)

dans laquelle:

- $X_0$, $X_1$, $X_2$, $X_3$, $X_4$, identiques ou différents, représentent chacun l'atome d'hydrogène, un halogène, un groupe alkyle inférieur, un groupe alkoxy inférieur, le groupe NRR' (où R et R', identiques ou différents, représentent chacun l'atome d'hydrogène ou un groupe alkyle inférieur), le groupe $NO_2$, $CF_3$ ou OH;
- $R_1$ représente l'atome d'hydrogène ou un groupe alkyle inférieur;
- $R_2$ représente l'atome d'hydrogène ou le groupe méthyle;
- Y représente un groupe OH, $OR_3$ (où $R_3$ est un groupe alkyle inférieur), NRR' (où R et R' sont définis comme ci-dessus) ou le groupe $O(CH_2)_nNR_4R_5$ (où n est un nombre entier ayant pour valeur 1 à 5 - et de préférence 2 ou 3 -; et $R_4$ et $R_5$, identiques ou différents, représentent chacun l'atome d'hydrogène, un groupe alkyle inférieur et peuvent former avec l'atome d'azote auquel ils sont liés un groupe hétérocyclique de 5 à 7 sommets susceptible d'être substitué et de comporter un ou plusieurs autres hétéroatomes tels que N et O);

(ii) leurs isomères géométriques; et
(iii) les sels des composés de formule I et de leurs isomères géométriques.

Par groupe alkyle inférieur on entend ici un reste hydrocarboné ramifié ou linéaire contenant 1 à 4 atomes de carbone comme par exemple les groupes méthyle, éthyle, propyle, isopropyle, n-butyle, isobutyle ou tertiobutyle. De même par groupe alkoxy inférieur on entend ici un groupe dont le radical hydrocarboné contient 1 à 4 atomes de carbone.

Par halogène on entend ici un atome de fluor, un atome de chlore, un atome de brome ou un atome d'iode, les atomes d'halogène préférés étant l'atome de fluor et de chlore.

Par cycle de 5 à 7 sommets pouvant être substitué et pouvant contenir éventuellement un ou plusieurs autres hétéroatomes on peut mentionner notamment les groupes pyrrolyle, imidazolyle, pyrazolyle, imidazolidinyle, pyrrolodinyle, pyrazolidinyle, pipéridyle, pipérazinyle, morpholinyle, homopipéridinyle, 4-méthylpipéridyle, 4-méthylpipérazinyle, 4-phénylpipérazinyle, 4-p-chlorophénylpipérazinyle et 4-β-hydroxyéthylpipérazinyle.

Les isomères géométriques des composés selon l'invention peuvent être de configuration «cis-cis», «trans-trans», «cis-trans» et «trans-cis». Les modalités opératoires décrites ci-dessous conduisent à des produits qui sont essentiellement de configuration «trans-trans» comme cela est démontré par RMN.

Par sels on entend ici (i) les sels minéraux obtenus à partir d'un acide de formule I (Y=OH) avec une base minérale (notamment NaOH, KOH, $NH_3$, $Ca(OH)_2$), (ii) les sels d'addition d'acide obtenus à partir d'un acide de formule I (Y=OH) avec une base organique et (iii) les sels d'addition d'acide obtenus à partir d'un composé de formule I présentant au moins un reste basique avec un acide minéral ou organique.

Les composés préférés sont les dérivés d'acide m-cinnamoyl-cinnamique tels que Y=OH, alkoxy en $C_1$-$C_4$, β-(4-méthylpipérazinyl)-éthoxy; $X_0$=H, $CH_3$; $X_1$=H, Cl, $OCH_3$; $X_2$=H, $OCH_3$; $X_3$=H, $OCH_3$; $X_4$=H, $OCH_3$; $R_1$=H ou $CH_3$ et $R_2$=H.

Le procédé de préparation que l'on préconise selon l'invention pour la préparation d'un composé de formule I est caractérisé en ce qu'on condense un dérivé d'acide acétyl-cinnamique de formule

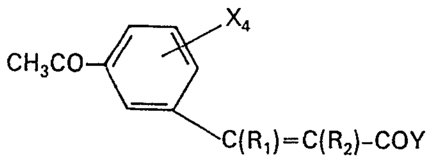

(IV)

(où $X_4$, Y, $R_1$ et $R_2$ sont définis comme ci-dessus) avec un benzaldéhyde de formule

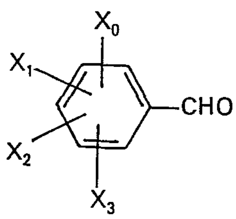

(V)

(où $X_0$, $X_1$, $X_2$ et $X_3$ sont définis comme ci-dessus), et en ce que, si nécessaire, on sépare les isomères géométriques.

De façon avantageuse, cette réaction est réalisée en faisant réagir IV et V dans une solution aqueuse d'un alcali (de préférence une solution aqueuse de NaOH à 400 g/l), ou dans un alcool inférieur en $C_1$–$C_4$ saturé par HCl gazeux. Cette technique convient parfaitement à la synthèse des acides I où Y est OH quand on opère en solution aqueuse en présence d'un alcali tel que la soude, à partir d'un acide ou d'un ester de formule IV. Quand on opère dans un alcool inférieur saturé en HCl gazeux, l'acide de formule IV donne l'acide de formule I et respectivement l'ester de formule IV donne l'ester de formule I; pour l'obtention d'un acide, l'alcool inférieur préféré est l'éthanol, et pour l'obtention d'un ester, l'alcool inférieur préféré est celui qui correspond au groupe ester du composé IV.

L'acide de formule I (Y=OH) peut être, le cas échéant, soumis à une réaction d'estérification ou d'amidification pour donner un ester ou une amide. De même l'ester de formule I (Y=alkoxy en $C_1$–$C_4$) peut être, le cas échéant, soumis à une réaction de saponification, de transestérification ou de transamidification pour donner un acide, un ester ou un amide.

La séparation des isomères géométriques peut être effectuée soit après la réaction de condensation, soit après les éventuelles saponifications, estérifications et amidifications envisagées ci-dessus. De préférence si cette séparation est jugée nécessaire elle sera mise en œuvre après la réaction de condensation, les isomères géométriques ainsi obtenus étant alors, le cas échéant, saponifiés, estérifiés ou amidifiés.

Les dérivés d'acide acétyl-cinnamique de formule IV [qui sont des produits intermédiaires nouveaux à l'exception des acides (Y=OH, $R_1$=$R_2$=$X_4$=H) et (Y=$OCH_3$, $X_4$=4-OH, et$R_1$=$R_2$=H) décrits dans CA 84, 164 457q et respectivement CA 89, 108 943 m] peuvent être préparés à partir d'un dérivé de 2-méthyl-2-phényl-1,3-dioxolanne de formule

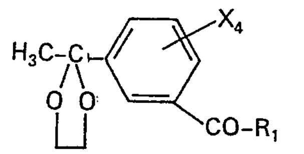

(II)

(où $X_4$ et $R_1$ sont définis comme ci-dessus).

Dans une première étape, on condense un composé de formule II au moyen

(a) soit d'un phosphonoalkanoate de triéthyle (et en particulier le phosphonacétate de triéthyle ou le phosphono-2 propionate de triéthyle) dans les conditions de la réaction de Horner-Emmons, en présence d'hydrure de sodium dans la diméthylformamide;

(b) soit d'acétate d'éthyle dans l'éthanol selon Claisen en présence de sodium dans le xylène; pour obtenir le composé III de formule

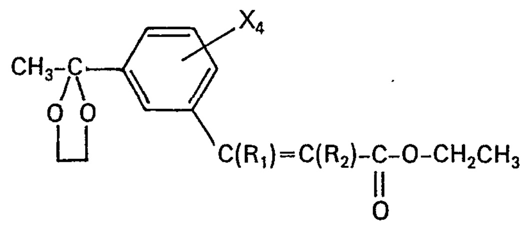

(III)

Puis, dans une seconde étape, on procède à la déprotection du groupe dioxolannyle au moyen d'un acide concentré (HCl 5N de préférence) puis, pour obtenir le produit IV utilisé comme matière première dans le procédé de l'invention, on procède par exemple à une saponification pour avoir l'acide IV, et à une estérification (ou transestérification) pour avoir l'ester IV.

On a donné ci-après des exemples nullement limitatifs de préparation de dérivés d'acide cinnamoyl-cinnamique de formule I.

Préparation I

Obtention de l'acide 3-[5-(p-chlorocinnamoyl)-2-méthoxyphényl]-but-2-ène-oïque.

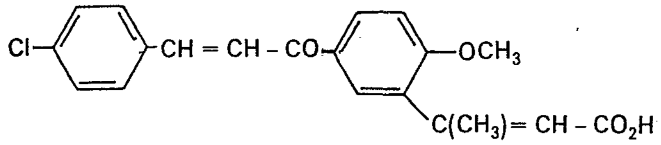

a. 3-[5-(1,1-Ethylènedioxy-éthyl)-2-méthoxyphényl]-but-2-ène-oate d'éthyle.

A 0°C, on ajoute goutte à goutte 0,24 mole (54 g) de phosphonacétate de triéthyle à 0,3 mole (7,2 g) d'hydrure de sodium en suspension dans 500 ml de N,N-diméthylformamide. On laisse revenir le milieu réactionnel à température ambiante (15–25°C) sous agitation. Après 5 h, le dégagement d'hydrogène est terminé. On porte alors le milieu réactionnel à −40°C et on additionne lentement 0,2 mole (47 g) de 5-(1,1-éthylènedioxy-éthyl)-2-méthoxy-acétophénone. On laisse revenir à température ambiante et on agite pendant 12 h puis on hydrolyse sur de l'eau glacée. Après extraction à l'éther, lavage des phases éthérées jusqu'à neutralité, séchage et évaporation du solvant, on recueille 45 g (rendement = 73%) du produit attendu qui se présente sous forme d'huile.

b. Acide 3-(5-acétyl-2-méthoxy-phényl)-but-2-ène-oïque.

45 g (0,147 mole) de l'ester précédent sont dissous dans 500 ml d'éthanol et 200 ml de soude à 10%. Après chauffage du mélange réactionnel 2 h à reflux, on laisse refroidir puis on acidifie à l'acide chlorhydrique 5N. On laisse précipiter le produit attendu et après filtration on le lave à l'eau puis à l'alcool. Par recristallisation dans 150 ml de méthanol, on obtient 15 g du produit désiré. F=194°C.

c. Acide 3-[5-(p-chlorocinnamoyl)-2-méthoxy-phényl]-but-2-ène-oïque.

4,7 g (0,02 mole) de l'acide précédemment obtenu sont dissous dans 30 ml de soude à 200 g/l. On ajoute au milieu réactionnel 2,8 g (0,02 mole) de para-chlorobenzaldéhyde et on agite pendant 2 h. Après acidification du mélange réactionnel le produit désire précipite. On filtre puis on lave le précipité à l'eau. Après recristallisation dans 300 ml d'éthanol on obtient 3 g de produit pur. F=215°C.

Préparation II

Obtention de l'acide 3-(5-cinnamoyl-2-méthoxyphényl)-but-2-ène-oïque.

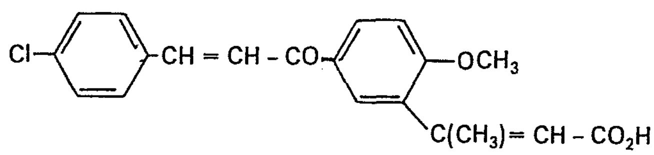

En procédant comme indiqué au stade c) de la préparation I, à partir de 1 g (4,25 millimoles) d'acide 3-(5-acétyl-2-méthoxy-phényl)-but-2-ène-oïque, 10 ml de soude à 200 g/l et 0,45 g (4,25 millimoles) de benzaldéhyde, on obtient 1,1 g du produit pur désiré F=190°C.

Préparation III

Obtention de l'acide 5-cinnamoyl-2-méthoxy-cinnamique.

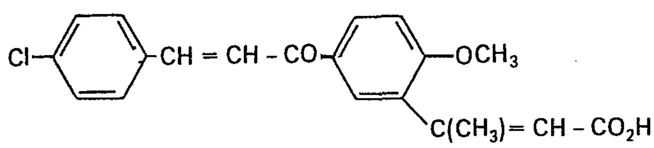

a. 3-[5-(1,1-Ethylènedioxy-éthyl)-2-méthoxy-phényl]-prop-2-ène-oate d'éthyle.

A 0°C, on ajoute goutte à goutte un mélange de 40 ml d'acetate d'éthyle et 1 cm³ d'éthanol à une suspension de 3,5 g (0,15 mole) de sodium en billes dans 200 ml de xylène. On ajoute ensuite 22 g (0,1 mole) de 5-(1,1-éthylènedioxy-éthyl)-2-méthoxy-benzaldéhyde dissous dans 50 ml de xylène. On laisse revenir le milieu réactionnel à température ambiante et on agite pendant 3 h. On additionne alors 50 ml d'acide acétique. Après disparition totale du sodium on ajoute 40 ml d'eau. Après extraction à l'éther, lavages à la soude puis à l'eau jusqu'à neutralité on évapore les phases éthérées pour obtenir 24 g (huile) du produit attendu.

b. 3-(5-Acétyl-2-méthoxyphényl)-prop-2-ène-oate d'éthyle.

On dissout 9,6 g de l'ester obtenu au stade a) dans 150 ml d'éther et on additionne 20 ml d'acide chlorhydrique 5N. On agite pendant 12 h. Après extraction par le mélange éther-$CH_2Cl_2$ (1:1) v/v puis lavages à l'eau, on obtient après évaporation des phases organiques 7,5 g du produit attendu. F=90°C.

c. Acide 3-(5-acétyl-2-méthoxyphényl)-prop-2-ène-oïque.

24 g (0,08 mole) de l'ester obtenu au stade b) sont dissous dans 250 ml de méthanol et 50 ml de soude à 400 g/l. Après chauffage du mélange réactionnel 2 h à reflux, on laisse refroidir puis on acidifie à l'acide chlorhydrique 5N. On laisse précipiter le produit attendu et, après filtration, on lave à l'eau puis à l'éthanol et enfin à l'éther isopropylique. On obtient 13,6 g du produit désiré. F=203°C.

d. Acide 3-(5-cinnamoyl-2-méthoxyphényl)-prop-2-ène-oïque.

Au départ de 10 g (0,045 mole) de l'acide obtenu au stade c), 80 ml de soude à 20% et 5 g (0,045 mole) de benzaldéhyde et selon un mode opératoire analogue à celui décrit au stade c) de la préparation I, on obtient 11,5 g du produit pur désiré. F=204°C.

Préparation IV

Obtention du 3-[m-(p-chlorocinnamoyl)-phényl]-prop-2-ène-oate d'éthyle.

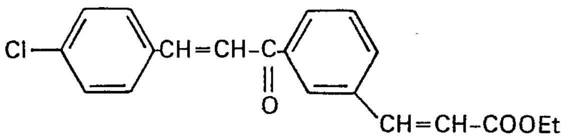

- autre nomenclature: m-(p-chlorocinnamoyl)-cinnamate d'éthyle -

On dissout 12 g (0,055 mole) de l'ester éthylique de l'acide 3-acétyl-cinnamique dans 120 ml d'éthanol. On additionne 8 g (0,057 mole) de para-chlorobenzaldéhyde puis 35 ml d'éthanol anhydre saturé d'acide chlorhydrique gazeux. On agite le milieu réactionnel pendant 10 h.

Par refroidissement à 0°C du milieu réactionnel, le produit recherché précipite. On filtre puis on lave à l'hexane. On obtient ainsi 13,5 g (rendement = 72%) du produit pur attendu. F=138°C.

De façon non limitative on a consigné dans le tableau I ci-après un certain nombre de composés selon l'invention.

Les produits selon l'invention sont utiles en thérapeutique en tant qu'agents favorisant la restauration d'un phénotype cellulaire de cellule normale chez une souche transformée. Ils peuvent être utilisés en particulier pour la prévention et le traitement des cancers, pour le traitement topique ou systémique des affections dermatologiques comme par exemple les kératoses cutanées, l'acné, le psoriasis, les eczémas, les verrues ou tout autre désordre dermatologique impliquant une altération du tissu corné et pour le traitement des altérations inflammatoires ou dégénératives des muqueuses, cartilages, muscles ou tendons, comme par exemple l'arthrose, les rhumatismes infectieux.

On rappelle que, quand on cultive des cellules normales (cellules saines) sur un milieu nutritif donné dans un espace donné, on observe qu'après un certain laps de temps que le nombre de cellules présentes est constant (confluence), et que quand on cultive dans les mêmes conditions des cellules transformées (cellules anormales ayant un développement anarchique) on n'obtient pas un nombre de cellules constant, la prolifération cellulaire ne cessant pas (absence de confluence).

On a résumé ci-après les résultats pharmacologiques obtenus sur un test de restauration de l'inhibition de croissance à confluence d'une culture de cellules transformées.

Le protocole opératoire utilisé est celui qui a été décrit par L.D. Dion et al. dans J. Natl. Cancer Inst. 58 (no 3), pages 795–801 (1977) et résumé ci-après. 20 000 cellules murines sont ensemencées sur une surface de 0,2 $cm^2$ sur un milieu de culture «Minimum Essential Medium» avec des sels de EAGLE et 10% de sérum de veau fœtal. Au bout de 4 h les cellules ont adhéré au support. Le milieu de culture est alors remplacé par le même milieu contenant le produit à tester dissous dans le DMSO.

A intervalle réguliers sur une période de 2 semaines, les cellules sont numérées après trypsination. La courbe de croissance obtenue est comparée à celle obtenue en l'absence de substance à tester.

L'effet de la drogue est évalué par la concentration minimale nécessaire pour inhiber toute croissance à partir du moment où la confluence est atteinte.

Les résultats obtenus ont été regroupés dans le tableau II ci-après, qui contient également les résultats relatifs à la toxicité (DL-0 ou DL-50) chez la souris par voie intrapéritonéale.

Un essai complémentaire a été entrepris avec le produit de l'exemple 9 selon le test d'inhibition de l'activité de l'ornithine-décarboxylase induite par le 12-O-tétradécanoylphorbol-13-acétate (en abrégé TPA) décrit par A.K. Verma et al. dans Cancer Research 38, 793–801 (1978).

Le produit en solution dans 100 µl d'acétone est testé par application topique à des souris, une heure avant application topique de 34 nmoles de TPA. On mesure l'activité de l'ornithine-décarboxylase 4,5 heures après le traitement au TPA. Dans ces conditions on obtient une inhibition de 48% de l'activité de l'ornithine-décarboxylase par administration topique de 34 nmoles du produit de l'exemple 9.

Selon l'invention, on préconise une composition thérapeutique, caractérisée en ce qu'elle renferme, en association avec un excipient physiologiquement acceptable, au moins un composé de formule I, un de ses isomères géométriques ou l'un de leurs sels pharmaceutiquement acceptables.

La posologie quotidienne que l'on préconise pour les composés selon l'invention est de 0,1 à 50 mg par kg de poids corporel (de préférence de 0,1 à 5 mg/kg), aous forme de gélules ou comprimés, pour administration orale.

Tableau I

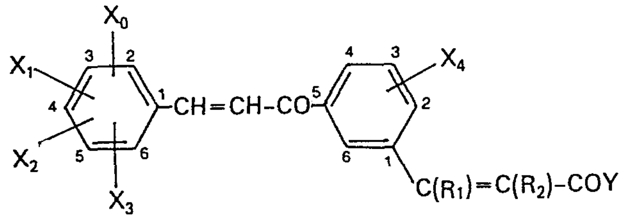

| Exemple | $X_0$ | $X_1$ | $X_2$ | $X_3$ | $X_4$ | $R_1$ | $R_2$ | Y | Point de fusion (°C) |
|---|---|---|---|---|---|---|---|---|---|
| 1 | H | 4-Cl | H | H | H | H | H | OH | 250 |
| 2 | H | 3-$OCH_3$ | 4-$OCH_3$ | 5-$OCH_3$ | H | H | H | $OC_2H_5$ | 133 |
| 3 | H | 4-Cl | H | H | H | H | H | $OC_2H_5$ | 138 |
| 4 | H | H | H | H | H | H | H | OH | 188 |
| 5 | H | H | H | H | 2-$OCH_3$ | $CH_3$ | H | OH | 190 |
| 6 | H | H | H | H | 2-$OCH_3$ | H | H | $O(CH_2)_2$- 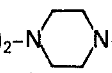 -$CH_3$ difumarate | 180 |
| 7 | H | H | H | H | 2-$OCH_3$ | H | H | OH | 209 |
| 8 | H | 4-$OCH_3$ | H | H | 2-$OCH_3$ | H | H | OH | 230 |
| 9 | H | 4-Cl | H | H | 2-$OCH_3$ | $CH_3$ | H | OH | 215 |
| 10 | H | 4-Cl | H | H | 2-$OCH_3$ | H | H | OH | 242 |
| 11 | H | 4-Cl | H | H | 2-$OCH_3$ | $CH_3$ | H | $NH_2$ | 199 |
| 12 | H | 4-$CH_3$ | H | H | 2-$OCH_3$ | $CH_3$ | H | OH | 210 |
| 13 | H | 4-Cl | H | H | 2-$OCH_3$ | H | $CH_3$ | OH | 222 |
| 14 | H | 3-$OCH_3$ | 4-$OCH_3$ | 5-$OCH_3$ | 2-$OCH_3$ | $CH_3$ | H | $OC_2H_5$ | 120 |
| 15 | H | 2-$CH_3$ | H | H | 2-$OCH_3$ | $CH_3$ | H | OH | 202 |
| 16 | H | 2-Cl | H | H | 2-$OCH_3$ | $CH_3$ | H | OH | 232 |
| 17 | H | 3-Cl | 4-Cl | H | 2-$OCH_3$ | $CH_3$ | H | OH | 212 |
| 18 | 2-$CH_3$ | 3-$CH_3$ | 4-$OCH_3$ | 6-$CH_3$ | 2-$OCH_3$ | $CH_3$ | H | OH | 117 |
| 19 | H | 4-Cl | H | H | 2-$OCH_3$ | $CH_3$ | H | $OC_2H_5$ | 129 |
| 20 | H | 4-Cl | H | H | 2-$OCH_3$ | $CH_3$ | H | -$O(CH_2)_2Net_2$ | 120 |
| 21 | H | 4-Cl | H | H | 2-$OCH_3$ | $CH_3$ | H | - 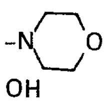 | 140 |
| 22 | H | 4-Cl | H | H | H | $CH_3$ | H | OH | 189 |

Rm la présence de la configuration «trans-trans» du produit de l'exemple 18 a été apportée par étude RMN qui donne:
- double liaison c=c du groupe chalcone $J_{H-H}$ - 16 Hz
- double liaison c=c du groupe cinnamique $J_{H-H}$ = 1,1 Hz

Tableau II

| Exemple | Toxicité i.p. souris (mg/kg) | Concentration d'inhibition de croissance à confluence en µg/ml* |
|---|---|---|
| 1 | DL-0 ⩾1600 | 5 à 10 |
| 2 | DL-0 ⩾1600 | 10 |
| 3 | DL-0 ⩾1600 | - |
| 4 | DL-50 = 450 | >10 |
| 5 | DL-50 = 550 | 5 à 10 |
| 6 | DL-50 = 300 | - |
| 7 | DL-50 =1000 | - |
| 8 | DL-0 ⩾ 800 | >10 |
| 9 | DL-0 ⩾1600 | 5 |

* Produit de référence: acide rétinoïque: 5 µg/ml

**Revendications pour les Etats contractants: BE, CH, LI, DE, FR, GB, IT, LU, NL, SE**

1. Dérivé d'acide cinnamoyl-cinnamique, caractérisé en ce qu'il est choisi parmi l'ensemble constitué par (i) les dérivés d'acide m-cinnamoyl-cinnamique de formule

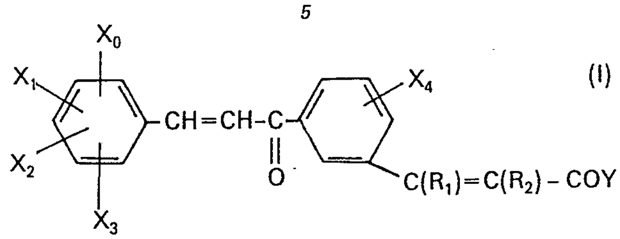

(I)

dans laquelle:
- $X_0$, $X_1$, $X_2$, $X_3$, $X_4$, identiques ou différents, représentent chacun l'atome d'hydrogène, un halogène, un groupe alkyle inférieur, un groupe alkoxy inférieur, le groupe NRR′ (où R et R′, identiques ou différents, représentent chacun l'atome d'hydrogène ou un groupe alkyle inférieur), le groupe $NO_2$, $CF_3$ ou OH;
- $R_1$ représente l'atome d'hydrogène ou un groupe alkyle inférieur;
- $R_2$ représente l'atome d'hydrogène ou le groupe méthyle;
- Y représente un groupe OH, $OR_3$ (où $R_3$ est un groupe alkyle inférieur), NRR′ (où R et R′ sont définis comme ci-dessus) ou le groupe $O(CH_2)_nNR_4R_5$ (où n est un nombre entier ayant pour valeur 1 à 5 - et de préférence 2 ou 3 -; et $R_4$ et $R_5$, identiques ou différents, représentent chacun l'atome d'hydrogène, un groupe alkyle inférieur, et peuvent former avec l'atome d'azote auquel ils sont liés un groupe hétérocyclique de 5 à 7 sommets susceptible d'être substitué et de comporter un ou plusieurs autres hétéroatomes tels que N et O);

(ii) leurs isomères géométriques; et
(iii) leurs sels.

2. Dérivé selon la revendication 1, caractérisé en ce que le groupe hétérocyclique $NR_4R_5$ est choisi parmi l'ensemble constitué par les groupes pyrrolyle, imidazolyle, pyrazolyle, imidazolidinyle, pyrrolidinyle, pyrazolidinyle, pipéridyle, pipérazinyle, morpholinyle, homopipéridinyle, 4-méthylpipéridyle, 4-méthylpipérazinyle, 4-phénylpipérazinyle, 4-p-chlorophénylpipérazinyle et 4-β-hydroxyéthylpipérazinyle.

3. Dérivé selon la revendication 1, caractérisé en ce que Y est OH, alkoxy en $C_1$-$C_4$, $NH_2$, $O(CH_2)_2N(C_2H_5)_2$, morpholino ou β-(4-méthylpipérazinyl)-éthoxy, $X_0$ est H ou $CH_3$, $X_1$ est H, Cl ou $OCH_3$, $X_2$ est H ou $OCH_3$, $X_3$ est H ou $OCH_3$, $X_4$ est H ou $OCH_3$, $R_1$ est H ou $CH_3$ et $R_2$ est H.

4. Acide 3-{5-[(2,3,6-triméthyl-4-méthoxy)cinnamoyl]-2-méthoxyphényl}-but-2-ène-oïque.

5. Acide 3-(5-cinnamoyl-2-méthoxyphényl)-but-2-ène-oïque.

6. Acide 3-[5-(p-chlorocinnamoyl)-2-méthoxyphényl]-but-2-ène-oïque.

7. Procédé de préparation d'un composé de formule I selon la revendication 1, ledit procédé étant caractérisé en ce qu'on condense un dérivé d'acide acétyl-cinnamique de formule

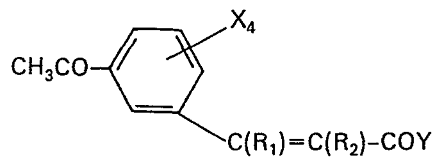

(IV)

(où $X_4$, Y, $R_1$ et $R_2$ sont définis comme ci-dessus) avec un benzaldéhyde de formule

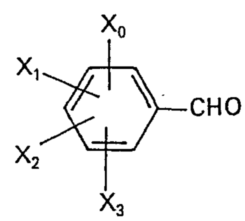

(V)

(où $X_0$, $X_1$, $X_2$ et $X_3$ sont définis comme ci-dessus), et en ce que, si nécessaire, on sépare les isomères géométriques.

8. Procédé selon la revendication 7 pour préparer un acide de formule I où Y est OH, caractérisé en ce que l'on fait réagir un dérivé d'acide acétyl-cinnamique IV (où Y est OH ou alkoxy en $C_1$-$C_4$) avec un benzaldéhyde V dans une solution aqueuse d'un alcali, de préférence une solution aqueuse de NaOH à 400 g/l.

9. Procédé selon la revendication 7 pour préparer un composé de formule I où Y est OH ou alkoxy en $C_1$-$C_4$, caractérisé en ce que l'on fait réagir un dérivé d'acide acétylcinnamique IV (où Y est OH ou alkoxy en $C_1$-$C_4$) avec un benzaldéhyde V dans un alcool inférieur en $C_1$-$C_4$ saturé en HCl gazeux, l'alcool en $C_1$-$C_4$ préféré pour l'obtention de l'ester Y = alkoxy en $C_1$-$C_4$ étant celui qui correspond au groupe ester du composé IV.

10. Compositon thérapeutique, caractérisée en ce qu'elle renferme, en association avec un excipient physiologiquement acceptable, au moins un dérivé d'acide cinnamoyl-cinnamique selon l'une quelconque des revendications 1 à 6 en tant qu'ingrédient actif.

**Revendications pour l'Etat contractant: AT**

1. Procédé de préparation de dérivés d'acide cinnamoyl-cinnamique de formule

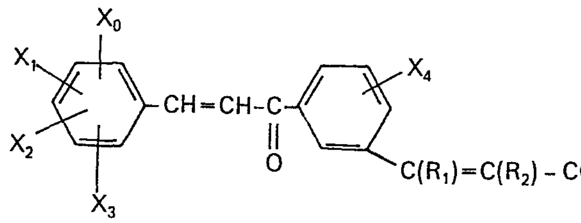

(I)

dans laquelle

- $X_0$, $X_1$, $X_2$, $X_3$, $X_4$, identiques ou différents, représentent chacun l'atome d'hydrogène, un halogène, un groupe alkyle inférieur, un groupe alkoxy inférieur, le groupe NRR' (où R et R', identiques ou différents, représentent chacun l'atome d'hydrogène ou un groupe alkyle inférieur), le groupe $NO_2$, $CF_3$ ou OH;
- $R_1$ représente l'atome d'hydrogène ou un groupe alkyle inférieur;
- $R_2$ représente l'atome d'hydrogène ou le groupe méthyle;
- Y représente un groupe OH, $OR_3$ (où $R_3$ est un groupe alkyle inférieur), NRR' (où R et R' sont définis comme ci-dessus) ou le groupe $O(CH_2)_nNR_4R_5$ (où n est un nombre entier ayant pour valeur 1 à 5 - et de préférence 2 ou 3 -; et $R_4$ et $R_5$, identiques ou différents, représentent chacun l'atome d'hydrogène, un groupe alkyle inférieur, et peuvent former avec l'atome d'azote auquel ils sont liés un groupe hétérocyclique de 5 à 7 sommets susceptible d'être substitué et de comporter un ou plusieurs autres hétéroatomes tels que N et O); leurs isomères géométriques et leurs sels, ledit procédé étant caractérisé en ce que l'on condense un dérivé d'acide acétyl-cinnamique de formule

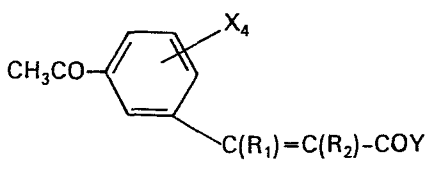

(IV)

(où $X_4$, Y, $R_1$ et $R_2$ sont définis comme ci-dessus) avec un benzaldéhyde de formule

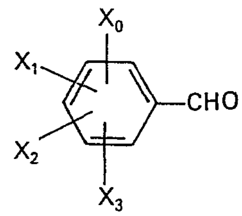

(V)

(où $X_0$, $X_1$, $X_2$ et $X_3$ sont définis comme ci-dessus), et en ce que, si nécessaire, on sépare les isomères géométriques.

2. Procédé selon la revendication 1 pour préparer un acide de formule I où Y est OH, caractérisé en ce que l'on fait réagir un dérivé d'acide acétyl-cinnamique IV (où Y est OH ou alkoxy en $C_1$-$C_4$) avec un benzaldéhyde V dans une solution aqueuse d'un alcali, de préférence une solution aqueuse de NaOH à 400 g/l.

3. Procédé selon la revendication 1 pour préparer un dérivé de formule I où Y est OH ou alkoxy en $C_1$-$C_4$, caractérisé en ce que l'on fait réagir un dérivé d'acide acétylcinnamique IV (où Y est OH ou alkoxy en $C_1$-$C_4$) avec un benzaldéhyde V dans un alcool inférieur en $C_1$-$C_4$ saturé en HCl gazeux, l'alcool en $C_1$-$C_4$ préféré pour l'obtention de l'ester Y = alkoxy en $C_1$-$C_4$ étant celui qui correspond au groupe ester du composé IV.

**Patentansprüche für die Vertragsstaaten: BE, CH, LI, DE, FR, GB, IT, LU, NL, SE**

1. Cinnamoylzimtsäurederivat, dadurch gekennzeichnet, dass es aus der Verbindungsgruppierung die aus

(i) den Derivaten der m-Cinnamoylzimtsäure mit der Formel

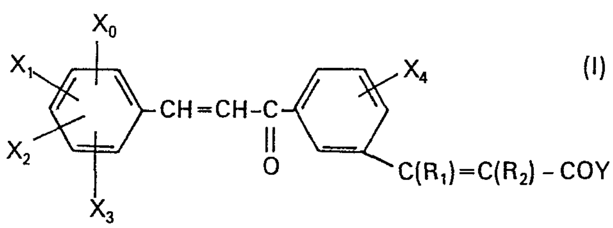

(I)

in der:

- $X_0$, $X_1$, $X_2$, $X_3$, $X_4$, identisch oder unterschiedlich, jeweils ein Wasserstoffatom, ein Halogen, eine niedere Alkylgruppe, eine NRR' Gruppe (wobei R und R', identisch oder unterschiedlich, jeweils ein Wasserstoffatom oder eine niedere Alkylgruppe bedeuten), eine $NO_2$, $CF_3$ oder OH Gruppe darstellen;

– $R_1$ ein Wasserstoffatom oder eine niedere Alkylgruppe darstellt;
– $R_2$ ein Wasserstoffatom oder eine Methylgruppe darstellt;
– Y eine OH, $OR_3$ (wobei $R_3$ eine niedere Alkylgruppe bedeutet), eine NRR′ Gruppe (wobei R und R′ wie oben definiert sind) oder eine $O(CH_2)_nNR_4R_5$ Gruppe (wobei n eine ganze Zahl ist, die den Wert 1 bis 5 – und vorzugsweise 2 oder 3 – haben kann, und wobei $R_4$ und $R_5$, identisch oder unterschiedlich, jeweils ein Wasserstoffatom oder eine niedere Alkylgruppe bedeuten und mit dem Stickstoffatom, an das sie gebunden sind, eine heterozyklische Gruppe mit 5 bis 7 Gliedern bilden, die substituiert werden kann und ein oder mehrere andere Heteroatome wie N und O enthalten kann) darstellt;

(ii) ihren geometrischen Isomeren; und

(iii) ihren Salzen;

besteht, gewählt wird.

2. Cinnamoylzimtsäurederivat gemäss Anspruch 1, dadurch gekennzeichnet, dass die $NR_4R_5$ heterozyklische Gruppe aus der Gruppierung, die aus Pyrrolyl-, Imidazolyl-, Pyrazolyl-, Imidazolinyl-, Pyrrolidinyl-, Pyrazolidinyl-, Piperidyl-, Piperazinyl-, Morpholinyl-, Homopiperidinyl-, 4-Methylpiperidyl-, 4-Methylpiperazinyl-, 4-Phenylpiperazinyl-, 4-p-Chlorophenylpiperazinyl-, und 4-β-Hydroxyäthylpiperazinylgruppen besteht, gewählt wird.

3. Cinnamoylzimtsäurederivat gemäss Anspruch 1, dadurch gekennzeichnet, dass Y OH, $C_1$–$C_4$-Alkoxy, $NH_2$, $O(CH_2)_2N(C_2H_5)_2$, morpholino oder β-(4-Methylpiperazinyl)-äthoxy ist, $X_0$ H oder $CH_3$ ist, $X_1$ H, Cl oder $CH_3$ ist, $X_2$ H oder $OCH_3$ ist, $X_3$ H oder $CH_3$ ist, $X_4$ H oder $OCH_3$ ist, $R_1$ H oder $CH_3$ ist, und $R_2$ H ist.

4. 3-{5-[(2,3,6-Trimethyl-4-methoxy)-cinnamoyl]-2-methoxyphenyl}-2-butensäure.

5. 3-(5-Cinnamoyl-2-methoxyphenyl)-2-butensäure.

6. 3-[5-(p-Chlorocinnamoyl)-2-methoxyphenyl]-2-butensäure.

7. Verfahren zur Herstellung einer Verbindung mit der Formel I nach Anspruch 1, dadurch gekennzeichnet, dass ein Derivat der Acetyl-zimtsäure der Formel

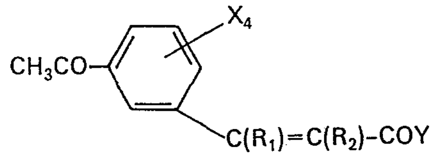

(IV)

(wobei $X_4$, Y, $R_1$ und $R_2$ wie oben definiert sind) mit einem Benzaldehyd der Formel

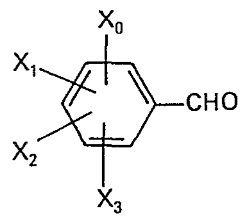

(V)

(wobei $X_0$, $X_1$, $X_2$ und $X_3$ wie oben definiert sind) kondensiert wird, und dadurch, dass die geometrischen Isomere, wenn es notwendig ist, getrennt werden.

8. Verfahren gemäss Anspruch 7 zur Herstellung einer Säure der Formel I, wobei Y OH ist, dadurch gekennzeichnet, dass ein Derivat der Acetyl-zimtsäure IV (wobei Y OH oder $C_1$–$C_4$-Alkoxy ist) mit einem Benzaldehyd V in einer wässrigen Lösung, die 400 g/l NaOH enthält, zur Reaktion gebracht wird.

9. Verfahren gemäss Anspruch 7 zur Herstellung einer Verbindung der Formel I, wobei Y OH oder $C_1$–$C_4$-Alkoxy ist, dadurch gekennzeichnet, dass ein Derivat der Acetyl-zimtsäure IV (wobei Y OH oder $C_1$–$C_4$-Alkoxy ist) mit einem Benzaldehyd V in einem niederen Alkohol in $C_1$–$C_4$, der mit gasförmigen HCl gesättigt ist, zur Reaktion gebracht wird, wobei der vorzugsweise zur Gewinnung des Esters Y = $C_1$–$C_4$-Alkoxy verwendete Alkohol in $C_1$–$C_4$ solcherart ist, dass er der Estergruppe der Verbindung IV entspricht.

10. Therapeutische Zusammensetzung, dadurch gekennzeichnet, dass sie, in Kombination mit einem physiologisch verträglichen Hilfsstoff, mindestens ein Cinnamoylzimtsäurederivat nach den Ansprüchen 1, 2, 3, 4, 5 oder 6 enthält.

**Patentansprüche für den Vertragsstaat: AT**

1. Verfahren zur Herstellung von Cinnamoylzimtsäurederivaten der Formel

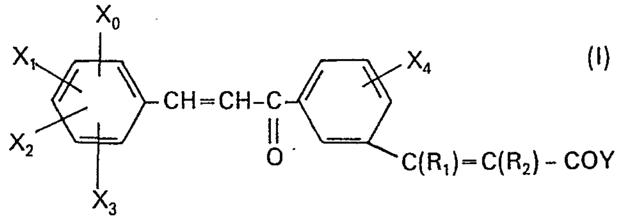

(I)

worin $X_0$, $X_1$, $X_2$, $X_3$, $X_4$, gleich oder verschieden, jeweils ein Wasserstoffatom, ein Halogen, eine Niedrigalkylgruppe, eine Niedrigalkoxygruppe, die Gruppe NRR′ (worin R und R′, gleich oder verschieden, jeweils ein Wasserstoffatom oder eine Niedrigalkylgruppe bedeuten), die Gruppe $NO_2$, $CF_3$ oder OH darstellen; $R_1$ ein Wasserstoffatom oder eine Niedrigalkylgruppe darstellt; $R_2$ ein Wasserstoffatom oder eine Methylgruppe darstellt; Y für eine OH-, $OR_3$- (worin $R_3$ eine Niedrigalkylgruppe ist), NRR′-Gruppe (worin R und R′ obige Bedeutung haben) oder die Gruppe

$O(CH_2)_nNR_4R_5$ (worin n eine ganze Zahl im Wert von 1 bis 5 – und vorzugsweise 2 oder 3 – ist; und $R_4$ und $R_5$, gleich oder verschieden, jeweils ein Wasserstoffatom, eine Niedrigalkylgruppe bedeuten und mit dem Stickstoffatom, an das sie gebunden sind, eine heterocyclische Gruppe mit 5 bis 7 Gliedern bilden, die substituiert werden kann und ein oder mehrere weitere Heteroatome, wie N und O, umfassen kann) steht; sowie ihrer geometrischen Isomeren und ihrer Salze, dadurch gekennzeichnet, dass ein Derivat der Acetylzimtsäure der Formel

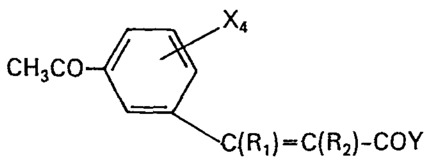

(IV)

worin $X_4$, Y, $R_1$ und $R_2$ obige Bedeutung haben, mit einem Benzaldehyd der Formel

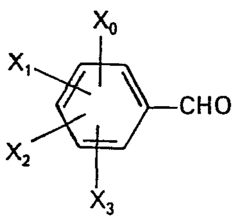

(V)

worin $X_0$, $X_1$, $X_2$ und $X_3$ obige Bedeutung haben, kondensiert wird und notwendigenfalls die geometrischen Isomeren voneinander getrennt werden.

2. Verfahren nach Anspruch 1 zur Herstellung einer Säure der Formel I, worin Y OH ist, dadurch gekennzeichnet, dass ein Derivat der Acetylzimtsäure IV (worin Y OH oder $C_1$–$C_4$-Alkoxy ist) mit einem Benzaldehyd V in einer wässrigen Lösung eines Alkalis, vorzugsweise einer wässerigen Lösung mit 400 g NaOH/l, zur Reaktion gebracht wird.

3. Verfahren nach Anspruch 1 zur Herstellung einer Verbindung der Formel I, worin Y OH oder $C_1$–$C_4$-Alkoxy ist, dadurch gekennzeichnet, dass ein Derivat der Acetylzimtsäure IV (worin Y OH oder $C_1$–$C_4$-Alkoxy ist) mit einem Benzaldehyd V in einem mit gasförmiger HCl gesättigten niedrigen $C_1$–$C_4$-Alkohol zur Reaktion gebracht wird, wobei der zur Gewinnung des Esters Y = $C_1$–$C_4$-Alkoxy bevorzugte $C_1$–$C_4$-Alkohol jener ist, der der Estergruppe der Verbindung IV entspricht.

**Claims for the Contracting States: BE, CH, LI, DE, FR, GB, IT, LU, NL, SE**

1. A cinnamoyl-cinnamic acid derivative, characterised in that it is selected from the group consisting of:

(i) m-cinnamoyl-cinnamic acid derivatives of the formula:

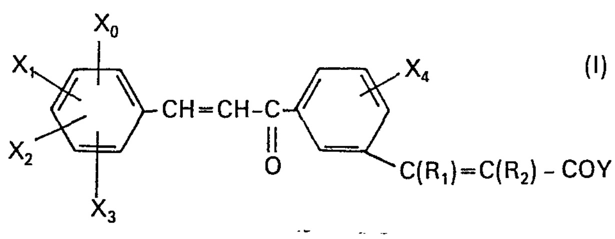

(I)

wherein:

- $X_0$, $X_1$, $X_2$, $X_3$, $X_4$, which are identical or different, each represent a hydrogen atom, a halogen, a lower alkyl group, a lower alkoxy group, a NRR′ group (wherein R and R′, which may be identical or different, each represent an atom of hydrogen or a lower alkyl group), a $NO_2$, $CF_3$ or OH group;
- $R_1$ represents a hydrogen atom or a lower alkyl group
- $R_2$ represents a hydrogen atom or $CH_3$;
- Y represents a group OH, $OR_3$ (wherein $R_3$ is a lower alkyl group), NRR′ (where R and R′ are defined as hereinabove) or the group $O(CH_2)_nNR_4R_5$ (wherein n is an integer of value 1 to 5 – and preferably 2 or 3 –; and $R_4$ and $R_5$, identical or different, each represent a hydrogen atom, a lower alkyl group and may form with the nitrogen atom to which they are bonded a heterocyclic group of 5 to 7 vertices capable of being substituted and of comprising one or more other heteroatoms such as N and O);

(ii) their geometrical isomers; and
(iii) their salts.

2. A derivative according to claim 1, characterised in that the heterocyclic group $NR_4R_5$ is selected from the group consisting of pyrrolyl, imidazolyl, pyrazolyl, imidazolidinyl, pyrrolidinyl, pyrazolidinyl, piperidyl, piperazinyl, morpholinyl, homopiperidinyl, 4-methylpiperidyl, 4-methylpiperazinyl, 4-phenylpiperazinyl, 4-p-chlorophenylpiperazinyl and 4-β-hydroxyethylpiperazinyl groups.

3. A derivative according to claim 1, characterised in that Y is OH, $C_1$-$C_4$-alkoxy, $NH_2$, $O(CH_2)_2N(C_2H_5)_2$, morpholino or β-(4-methylpiperazinyl)-ethoxy, $X_0$ is H or $CH_3$, $X_1$ is H, Cl or $OCH_3$, $X_2$ is H or $OCH_3$, $X_3$ is H or $OCH_3$, $X_4$ is H or $OCH_3$, $R_1$ is H or $CH_3$ and $R_2$ is H.

4. 3-{5-[(2,3,6-Trimethyl-4-methoxy)-cinnamoyl]-2-methoxyphenyl}-but-2-ene-oic acid.

5. 3-(5-Cinnamoyl-2-methoxyphenyl)-but-2-ene-oic acid.

6. 3-[5-(p-Chlorocinnamoyl)-2-methoxyphenyl]-but-2-ene-oic acid.

7. A method for preparing a compound of formula I according to claim 1, said method being characterised in that an acetyl cinnamic acid derivative of the formula:

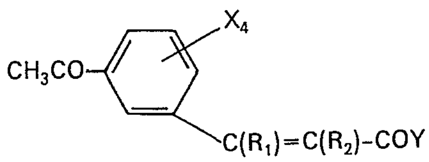

(IV)

(wherein $X_4$, Y, $R_1$ and $R_2$ are defined as hereinabove) is condensed with a benzaldehyde of the formula:

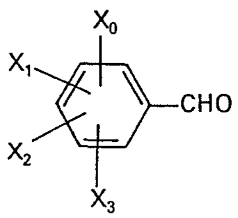

(V)

(wherein $X_0$, $X_1$, $X_2$ and $X_3$ are defined as hereinabove), and in that, if necessary, the geometrical isomers are separated.

8. A method according to claim 7 for preparing an acid of formula I wherein Y is OH, characterised in that an acetyl-cinnamic acid derivative IV (wherein Y is OH or $C_1$-$C_4$-alkoxy) is reacted with a benzaldehyde V in an aqueous solution of an alkali, preferably an aqueous solution of NaOH at 400 g/l.

9. A method according to claim 7, for preparing a compound of formula I wherein Y is OH or $C_1$–$C_4$-alkoxy, characterised in that an acetyl-cinnamic acid derivative IV (wherein Y is OH or $C_1$–$C_4$-alkoxy) is reacted with a benzaldehyde V in a lower $C_1$–$C_4$-alcohol saturated in gaseous HCl, the preferred $C_1$–$C_4$-alcohol for obtaining the ester Y = $C_1$–$C_4$-alkoxy being the one corresponding to the ester group of compound IV.

10. A therapeutical composition, characterised in that it contains, in association with a physiologically acceptable excipient, at least one cinnamoyl-cinnamic acid derivative according to any one of claims 1 to 6, as active ingredient.

**Claims for the contracting State: AT**

1. A method for preparing cinnamoyl-cinnamic acid derivatives of the formula

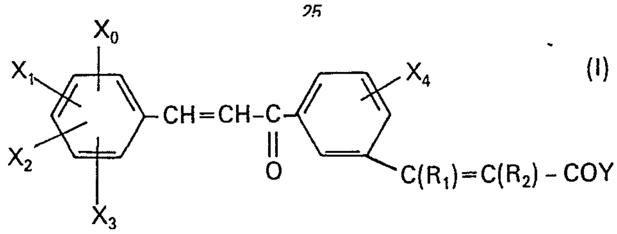

(I)

wherein:

- $X_0$, $X_1$, $X_2$, $X_3$, $X_4$, which are identical or different, each represent a hydrogen atom, a halogen, a lower alkyl group, a lower alkoxy group, a NRR′ group (wherein R and R′, which may be identical or different, each represent an atom of hydrogen or a lower alkyl group), a $NO_2$, $CF_3$ or OH group;
- $R_1$ represents a hydrogen atom or a lower alkyl group
- $R_2$ represents a hydrogen atom or $CH_3$;
- Y represents a group OH, $OR_3$ (wherein $R_3$ is a lower alkyl group), NRR′ (where R and R′ are defined as hereinabove) or the group $O(CH_2)_nNR_4R_5$ (wherein n is an integer of value 1 to 5 – and preferably 2 or 3 –; and $R_4$ and $R_5$, identical or different, each represent a hydrogen atom, a lower alkyl group and may form with the nitrogen atom to which they are bonded a heterocyclic group of 5 to 7 vertices capable of being substituted and of comprising one or more other heteroatoms such as N and O); their geometrical isomers and their salts, said method being characterised in that an acetyl-cinnamic acid derivative of the formula:

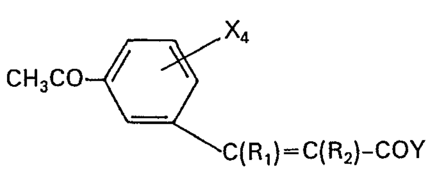

(IV)

(wherein $X_4$, Y, $R_1$ and $R_2$ are defined as hereinabove) is condensed with a benzaldehyde of the formula;

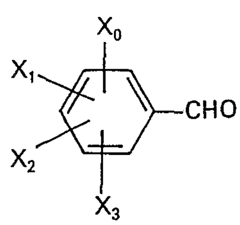

(V)

(wherein $X_0$, $X_1$, $X_2$ and $X_3$ are defined as hereinabove), and in that, if necessary, the geometrical isomers are separated.

2. A method according to claim 1 for preparing an acid of formula I wherein Y is OH, characterised in that an acetyl-cinnamic acid derivative IV (wherein Y is OH or $C_1$-$C_4$-alkoxy) is reacted with a benzaldehyde V in an aqueous solution of an alkali, preferably an aqueous solution of NaOH at 400 g/l.

3. A method according to claim 1, for preparing a compound of formula I wherein Y is OH or $C_1$–$C_4$-alkoxy, characterised in that an acetyl-cinnamic acid derivative IV (wherein Y is OH or $C_1$–$C_4$-alkoxy) is reacted with a benzaldehyde V in a lower $C_1$–$C_4$-alcohol saturated in gazeous HCl, the preferred $C_1$–$C_4$-alcohol for obtaining the ester Y = $C_1$–$C_4$-alkoxy being the one corresponding to the ester group of compound IV.